# EUROPEAN PATENT SPECIFICATION

(11) **EP 1 941 269 B1**
(45) Date of publication and mention of the grant of the patent: **21.10.2015**
(21) Application number: 06813046.7
(22) Date of filing: 25.10.2006
(51) Int. Cl.: G01N 33/543, G01N 30/88, G01N 30/02

(54) **A METHOD OF SCREENING A BIOLOGICAL TARGET FOR WEAK INTERACTIONS USING WEAK AFFINITY CHROMATOGRAPHY**
VERFAHREN ZUM SCREENING EINES BIOLOGISCHEN ZIELS AUF SCHWACHE WECHSELWIRKUNGEN UNTER VERWENDUNG SCHWACHER AFFINITÄTSCHROMATOGRAPHIE
METHODE DE CRIBLAGE D'INTERACTIONS FAIBLES DANS UNE CIBLE BIOLOGIQUE, FAISANT APPEL A UNE CHROMATOGRAPHIE D'AFFINITE FAIBLE

(30) Priority: 27.10.2005 US 730808 P
(43) Date of publication of application: 09.07.2008
(73) Proprietor: Transientic Interactions AB, 590 95 Loftahammar (SE)
(72) Inventor: OHLSON, Sten, S-211 16 Malmö (SE); SHORAVI, Siamak, S-393 50 Kalmar (SE); FEX, Tomas, S-226 52 Lund (SE); ISAKSSON, Roland, S-224 71 Lund (SE)
(74) Representative: Holmberg, Martin Tor
(86) International application number: PCT/SE2006/050419
(87) International publication number: WO 2007/050032

(56) References cited:
- EP-A- 0 290 406
- WO-A1-91/12820
- OHLSON S ET AL: "Use of monoclonal antibodies for weak affinity chromatography" JOURNAL OF CHROMATOGRAPHY, ELSEVIER SCIENCE PUBLISHERS B.V. AMSTERDAM, NL, vol. 758, no. 2, 17 January 1997 (1997-01-17), pages 199-208, XP004034191 ISSN: 0021-9673
- LEICKT L ET AL: "Affinity screening for weak monoclonal antibodies" JOURNAL OF IMMUNOLOGICAL METHODS, ELSEVIER SCIENCE PUBLISHERS B.V.,AMSTERDAM, NL, vol. 220, no. 1-2, 1 November 1998 (1998-11-01), pages 19-24, XP004144098 ISSN: 0022-1759
- SARASWAT L D ET AL: "Affinity Ligand Selection from a Small Molecules: Assay Development Screening, and Application" BIOTECHNOLOGY PROGRESS, AMERICAN INSTITUTE OF CHEMICAL ENGINEERS, US, vol. 21, no. 1, 1 January 2005 (2005-01-01), pages 300-308, XP003009335 ISSN: 8756-7938
- LEICKT L. ET AL.: 'Affinity screening for weak monoclonal antibodies' JOURNAL OF IMMUNOLOGICAL METHODS vol. 220, 1998, pages 19 - 24, XP004144098
- SARASWAT L.D. ET AL.: 'Affinity Ligand Selection from a Small Molecules: Assay Development Screening, and Application' BIOTECHNOL. PROG. vol. 21, 2005, pages 300 - 308, XP003009335
- OHLSON S. ET AL.: 'Screening for transient biological interactions as applied to albumin ligands: A new concept for drug discovery' ANALYTICAL BIOCHEMISTRY vol. 359, 2006, pages 120 - 123, XP005723847
- OHLSON S. ET AL.: 'Novel Approach to Affinity Chromatography Using Weak' ANALYTICAL BIOCHEMISTRY vol. 169, 1988, pages 204 - 208, XP003009336
- ZOPF D. ET AL.: 'Weak-affinity chromatography' NATURE vol. 346, 05 July 1990, pages 87 - 88, XP003009337
- Strandh, Magnus: "Insights into Weak Affinity Antibody-Antigen Interactions", , 2000, Retrieved from the Internet: URL:http://www.immun.lth.se/fileadmin/immu n/Avhandlingar/Magnus_Strandh.pdf [retrieved on 2012-05-25]

## Description

### Background of invention

The notion that many biological interactions are based on transient binding (dissociation constants (K_{d}) in the range of 10 mM to 0.01 mM) is familiar, yet the implications for biological sciences have been realized only recently (Gabius, H.-J. & Gabius, S. (eds.) Glycosciences: Status and Perspectives (Chapman & Hall, Weinheim, 1997)). An important area of biological sciences is drug design where the traditional 'lock and key' view of binding has prevailed and drug candidates are usually selected on their merits as being tight binders. However, the rationale that transient interactions are of importance for drug discovery is slowly gaining acceptance. These interactions may relate not only to the desired target interaction, but also to unwanted interactions creating toxicity problems or to interactions with drug carriers involved in absorption and/or excretion (Steffansen, B. et al. Intestinal Solute Carriers: An Overview of Trends and Strategies for Improving Oral Drug Absorption. Eur. J. Pharm. Sciences 21, 3-16 (2004)). Here we demonstrate, in a high-throughput screening format, affinity selection of weak binders to a model target of albumin by zonal retardation chromatography (Steffansen, B. et al. Intestinal Solute Carriers: An Overview of Trends and Strategies for Improving Oral Drug Absorption. Eur. J. Pharm. Sciences 21, 3-16 (2004)). It is perceived that this approach can define the 'transient drug' as a complement to current drug discovery procedures.

Transient biological interactions form the essence of the interactome and occur constantly inside the cells, on cell surfaces or in the extra cellular matrix. They are 'dynamic', with dissociation rate constants (k_{d}) > 0.1 s⁻¹ and are governed by the local concentrations of the interacting biological pair. They can act in parallel, such as the interactions of the stacked bases of DNA, or in a serial fashion such as the action of small molecule substrates or inhibitors upon an enzyme. Biological macromolecules undergo a number of transient interactions through their component amino acids, carbohydrates, lipids or nucleotides. These macromolecules form assemblies expressed as cellular organelles which are involved in a complex interplay of weak interactions forming the network of the biological entity.

As technologies are evolving to study and characterize transient interactions based on chromatography (Strandh, M., Andersson, H. & Ohlson, S. in Methods in Molecular Biology (eds. Bailon, P., Ehrlich, G. K., Fung, W. J. & Berthold, W.) 7-24 (Humana Press, Inc, Totowa, NJ, 2000)), electrophoresis (Nilsson, M. et al. Determination of Protein-Ligand Affinity Constants from Direct Migration Time in Capillary Electrophoresis. Electrophoresis 25, 1829-1836 (2004)), surface plasmon resonance (SPR) biosensor (MacKenzie, C. R. et al. Analysis by Surface Plasmon Resonance of the Influence of Valence on the Ligand Binding Affinity and Kinetics of an Anti-Carbohydrate Antibody. The Journal of Biological Chemistry 271, 1527-1533 (1996)), fluorescence spectroscopy (Engström, H. A., Andersson, P. O. & Ohlson, S. Analysis of the Specificity and Thermodynamics of the Interaction between Low Affinity Antibodies and Carbohydrate Antigens using Fluorescence Spectroscopy. J Immunol Methods 297, 203-211 (2005)), nuclear magnetic resonance (NMR) (Pellechia, M., Sem, D. S. & Wuthrich, K. NMR in Drug Discovery. Nature Reviews in Drug Discovery 1, 211-219 (2002)), calorimetry (Plotnikov, V. et al. An Autosampling Differential Scanning Calorimeter Instrument for Studying Molecular Interactions. Assay Drug Dev. Technol. 1, 83-90 (2002)) and chemiluminescence (Causey, L. D. & Dwyer, D. S. Detection of Low Affinity Interactions Between Peptides and Heat Shock Proteins by Chemiluminescence of Enhanced Avidity Reactions (CLEAR). Nature Biotechnology 14, 348-351 (1996)), we are now gradually unraveling the nature of transient binding in various areas such as bacterial and viral interactions (Mammen, M., Choi, S.-K. & Whitesides, G. M. Polyvalent interactions in biological systems: Implications for design and use of multivalent ligands and inhibitors. Angew. Chem. Int. Ed. 37, 2754-2794 (1998)), cellular interactions (Dustin, M. L. et al. Low Affinity Interaction of Human or Rat T Cell Adhesion Molecule CD2 with Its Ligand Aligns Adhering Membranes to Achieve High Physiological Affinity. The Journal of Biological Chemistry 272, 30889-30898 (1997)), protein-protein (peptide) interactions (Karjalainen, K. High-Sensitivity, Low Affinity-Paradox of T-Cell Receptor Recognition. Current Opinion in Immunology 6, 9-12 (1994); Beeson, C. et al. Early Biochemical Signals Arise from Low Affinity TCR-Ligand Reactions as the Cell-Cell Interface. J. Exp. Med 184, 777-782 (1996); and Garcia, C. D., DeGail, J. H., Wilson, W. W. & Henry, C. S. Measuring Protein Interactions by Microchip Self-Interaction Chromatography. Biotechnol. Prog. 19, 1006-1010 (2003)), protein-carbohydrate interactions (Zopf, D. & Roth, S. Oligosaccharide Anti-infective Agents. Lancet 347, 1017-1021 (1996); and Elgavish, S. & Shaanan, B. Lectin-Carbohydrate interactions: Different Folds, Common Recognition Principles. TIBS 22, 462-467 (1997)) and carbohydrate-carbohydrate interactions (Fuente, J. M. d. 1. & Penadés, S. Understanding Carbohydrate-Carbohydrate Interactions by means of Glyconanotechnology. Glycoconjugate Journal 21, 149-163 (2004)).

The very nature of transient biological interactions makes them attractive in the laboratory for at least two major lines of investigation. First, as demonstrated in recent years, they can be used in diagnostic applications to continuously monitor fluctuating amounts of biomolecules in real-time (Ohlson, S., Jungar, C., Strandh, M. & Mandenius, C.-F. Continuous Weak-affinity Immunosensing. Trends in Biotechnology 18, 49-52 (2000)) or to develop more specific recognition molecules to targets where traditional approaches of high-affinity ligands may not offer a viable solution (Regenmortel, M. H. V. V. From absolute to exquisite specificity. Reflections on the fuzzy nature of species, specificity and antigenic sites. J. Immunol. Methods 216, 37-48 (1998); and Leickt, L., Grubb, A. & Ohlson, S. Development of Monoclonal Antibodies against creatine kinase CKMB2. Scand J Clin Lab Invest 62, 423-430 (2002)). Second, due to the transient nature of many interactions in a biological entity, it can be envisaged that synthetic drugs can be similar in terms of binding behaviour to their natural counterparts. Such 'transient' drugs can be attractive for a number of reasons. For example, they can, either in a monovalent or a polyvalent format, be designed with specificity superior to that of the traditional high affinity drug. Tolerance or severe side effects of drug action may be minimized with a weakly binding drug e.g. in its binding to cytochrome p450 proteins. Drug absorption can be increased through weak interactions with specific uptake mechanisms. Also the active concentration may be an efficient tool to govern the biological effect of the drug.

Furthermore, in the race for new drug molecules, there is increased interest to search compound libraries with small molecules which can subsequently be optimized by increasing size and introducing additional functionalities. Such fragment based approaches (Erlanson, D. A., McDowell, R. S. & O'Brien, T. Fragment-Based Drug Discovery. Journal of Medicinal Chemistry 47, 3463-3482 (2004)), which identify small molecules that bind with low affinity, are being used increasingly in drug discovery. Weak-binding drug candidate molecules in practice often are difficult to screen for. There are methods available based on NMR, mass spectrometry and X-ray crystallography but these are not designed to detect weak binders on a high throughput basis, where thousands of samples can be screened per day. In addition 'virtual screening' methods have also been applied with limited success, mainly with rigid inflexible fragments (Erickson, J. A., Jalaie, M., Robertson, D. H., Lewis, R. A. & Vieth, M. Lessons in Molecular Recognition: The Effects of Ligand and Protein Flexibility on Docking Accuracy. Journal of Medicinal Chemistry 47, 45-55 (2004)).

In recent years high throughput screening (HTS) of compound libraries has become an important tool for identification of potential drug candidates (Sundberg, S. A. High-Throughput and Ultra-High-Throughput Screening: Solution- and Cell-Based Approaches. Current Opinion in Biotechnology 11, 47-53 (2000); and Williams, G. P. Advances in High Throughput Screening. Drug Discovery Today 9, 515-516 (2004)). The HTS technology has evolved rapidly with the development of automated and miniaturized equipment that now can handle several hundred thousand samples. Standard-plate binding assay techniques are typically used with fluorescence, radioactivity, or optical absorbance as the detection platform. One important restriction with these techniques is that they can only measure strong interactions (a typical assay concentration is 10 µM) because transient drug binders are washed away in the assay procedure. Another limitation is that they only offer evidence of binding where identification of the binding partner has to be implemented.

Affinity chromatography has developed into a powerful tool mainly for the purification of proteins. In addition frontal affinity chromatography in combination with mass spectrometry has been used for drug discovery applications (Chan, N. W. C., Lewis, D. F., Rosner, P. J., Kelly, M. A. & Schriemer, D. C. Frontal Affinity Chromatography-Mass Spectrometry Assay Technology for Multiple Stages of Drug Discovery: Applications of a Chromatographic Biosensor. Anal Chem 319, 1-12 (2003)) for detecting high-affinity binders. Now, with the introduction of weak affinity chromatography (WAC) (Ohlson, S., Lundblad, A. & Zopf, D. Novel Approach to Affinity Chromatography Using "Weak" Monoclonal Antibodies. Analytical Biochemistry 169, 204-208 (1988)) for zonal retardation, high performance methods are emerging which provide information about affinity and kinetics of weak interactions with biomolecules, wherein chromatographic separation is usually carried out under mild isocratic conditions, which could be physiologically relevant. In order to efficiently identify candidates exhibiting weak affinity, there is a need for a methodology that supports rapid screening and selection of potential drug molecules. The present invention aims at providing such a method.

### Description of the invention

The present invention relates to a method of identifying candidate drug molecules exhibiting weak affinity by screening a biological target for transient weak interactions between the target and a library of ligands. The inventive method comprises the steps of providing a composition of a biological target; providing a plurality of stationary phases from said composition; transporting a plurality of ligand compositions to said stationary phases, thereby establishing contacts between said ligands and said biological targets; collecting, downstream of said stationary phases, zonal retardation information for each ligand; and finally selecting ligands exhibiting weak affinity to said target, wherein said ligands have dissociation constants (K_{d}) in the range of about 0.01 to about 10 mM. The selected ligands having transient bindings to the biological target may undergo further studies regarding their binding behaviour, for example with NMR analysis, with the purpose of identifying one or several lead compound(s). The method typically involves a detection step, wherein ligands arriving from said stationary phases during a time period sufficient to discriminate between different ligand affinities and for this purpose collecting zonal retardation information, most importantly retention time and bandwidth to estimate the affinity and the dynamics of each ligand/target interaction. The nature of the detection process is not critical for the invention and is rather determined with respect to the chemistry of the ligand library.

According to one aspect, the biological target is provided an immobilized composition, for example the target is immobilized to a solid support. The person skilled in this technology knows a number of suitable support materials and means to adsorb or chemically link biological molecules thereto. In one example, the immobilized composition may be used with chromatography columns or multi-well systems and means for elution with a mobile phase. In a specific example, a proteinaceous target can be immobilized to functionalized silica particles with conventional methods and packaged into a plurality of chromatography wells, whereupon ligand compositions are injected in each well before elution with mobile phase and collection of fractions for detection.

According to another aspect, stationary phases including a biological target can be formed in a in a plurality of miniaturized channels in a solid support, such as thin plate, a chip or a disc. The solid support may have a first zone for receiving a plurality of ligand compositions for transportation to said biological target and a detection zone downstream of said first zone. Suitably, the transportation in the solid support takes place by capillary forces and/or centrifugal forces when ligands travel from the first zone to the detection zone.

In an example on how the inventive method can practically be realized, a system is formed which includes a multi-well plate with immobilized biological target in each of the wells. The system further comprises means for supplying compositions of the ligands to each well and a series of matching collector plates for collecting fractions of eluted mobile phase and subsequent detection. The system may further comprise means to assist with supplementation, transportation and elution of the mobile phase. The following part of the specification provides more detailed and illustrative examples on how conduct the invention and the skilled person may be able to deduce a number of alternatives that still will fall under the appended claims.

### Detailed and exemplifying description of the invention

**Figure 1****:** Schematic set-up of zonal affinity separation screening in a 96-well format.
**Figure 2****:** Zonal weak affinity chromatography in a well. Bupivacain (1 mM) and propranolol (0.25 mM) were retarded as indicated by their peak apex. Sodium azide (0.5 mM) was a marker of void volume (non-retarded substance).
**Figure 3****:** Well-to-well reproducibility of zonal weak affinity chromatography of bupivacain (1 mM). Bupivacain was retarded in three different wells and chromatography was repeated once in each well.

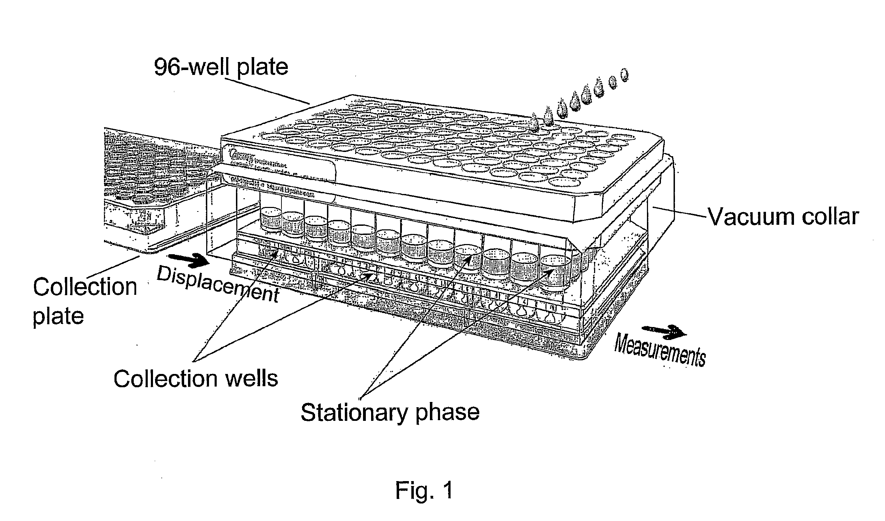

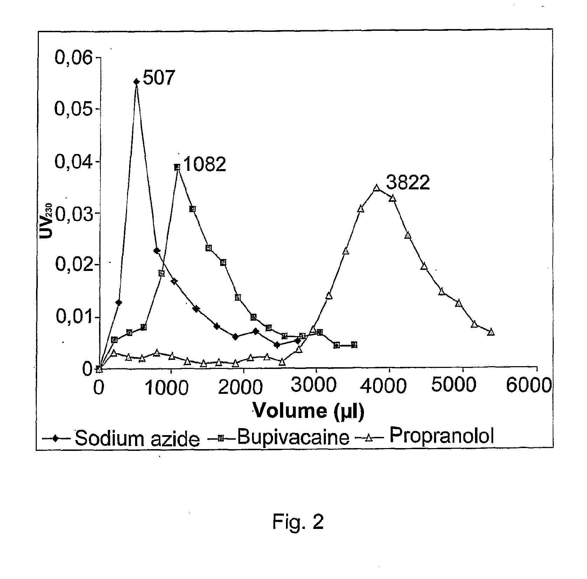

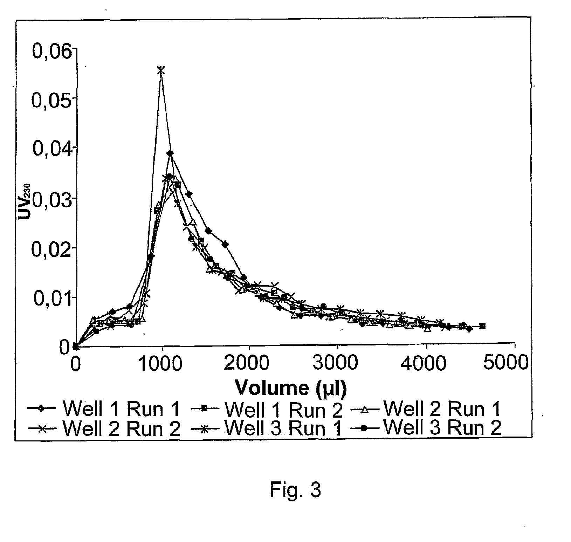

In this model study we have looked at the weak interactions between some known drugs and bovine serum albumin (BSA). Proteins like orosomucoid, bovine albumin and human albumin have successfully been utilized as selectors to determine the enantiomeric composition of a large number of drugs. Here the affinity between drug and protein typically fall in the range of a transient interaction i.e. K_{d}-values of 10⁻¹ - 10⁻⁵ M. It is well-known from earlier studies that BSA is an excellent model protein and shows variable binding affinity as well as (enantio)selectivity to a large number of drugs (Haginaka, J. Protein-based Chiral Stationary Phases for High-performance Liquid Chromatography Enantioseparations. J of Chromatography A 906, 253-273 (2001)). Even with the somewhat basic experimental set-up in a 96-well format (Figure 1), our results demonstrate (Figure 2) that small selectivity differences can be detected in spite of the short retention times, indicating its potential use for HTS applications. Substances are slightly retarded through transient binding, probably with affinities of 0.1-1 mM, as a consequence of high active concentration of albumin binding sites (approximately 1 mM). Furthermore the separations show excellent reproducibility as can be seen in Figure 3. The experiments also demonstrate the potential to run weak affinity separations in a parallel mode and in a multi-well plate format. With optimization of column packing techniques and liquid handling methods the relatively large volumes of the eluted zones (bands) will likely be reduced. The retention differences between propranolol, bupivacaine and sodium azide (giving the non-retained elution volume) are quite significant and in agreement with high performance liquid chromatography methods using corresponding commercial analytical columns.

The results presented here indicate the potential to use chromatography for screening of transient binding phenomena. By robotization and miniaturization of this analytical platform we anticipate that thousand of separations can be executed in one run in a matter of minutes. The results provide instant information about affinity and kinetics of relevant transient binders in terms of their retention time and band spreading. With the results above, we are convinced that the approach presented in this letter may offer a complementary tool that may open up alternative areas of drug research, looking for 'transient drugs' that bind dynamically to their targets. By implementing an HTS platform based on weak affinity chromatography, we will be able to explore this hypothesis in greater detail and to determine how this concept for drug discovery can be exploited

### Methods

### Preparation of BSA-silica material:

Diol microparticulate silica (10 m particles, pore size 300 A) was prepared according to standard methods (Ohlson, S., Lundblad, A. & Zopf, D. Novel Approach to Affinity Chromatography Using "Weak" Monoclonal Antibodies. Analytical Biochemistry 169, 204-208 (1988)) using γ-glycidoxipropyltrimethoxysilane as the silanization reagent. In a subsequent step, the diol phase was oxidized using periodic acid (H₅IO₆) to produce the aldehyde silica. To this support material, bovine serum albumin (BSA) was immobilized by reductive amination using sodiumcyanoborohydride to reduce the Schiff's base intermediate (BSA-diol-silica) (Ohlson, S., Lundblad, A. & Zopf, D. Novel Approach to Affinity Chromatography Using "Weak" Monoclonal Antibodies. Analytical Biochemistry 169, 204-208 (1988)). According to UV-analysis (280 nm) directly on gel, 130 mg BSA per g silica was immobilized corresponding to a yield of 80%.

### Preparation and packing of chromatography wells:

200 mg of reference gel (diol-silica) and assay gel (BSA-diol-silica) were suspended in 1 ml methanol and packed into each well in a 96-well filtering system (Captiva^{®} 96-well 10µ glassfiber filter plate from Ansys Technologies (Company), see figure 1) using vacuum applied to the wells. The gel in the wells was first washed with 5 ml MeOH and then with 5 ml 0.1 M sodium phosphate buffer pH 7.0. Filter discs were placed on top of the gel material in each well.

### Zonal weak affinity chromatography in a 96-well plate format: (See figure 1)

Wells containing gel materials were washed carefully with 0.05 M sodium phosphate buffer pH 7.0 using vacuum collar to remove non-bound BSA, contaminants and entrapped air. The excess of buffer was gently removed from the surface of the wells using pipette and 10 µl of test substances were injected centrally in each wells which were then flushed with mobile phase (0.05 M sodium phosphate buffer pH 7.0). Vacuum was applied to the collar and the mobile phase was flowed into a 96 well collection plate (Nunclon, Nalgene-Nunc) within 8-10 seconds. Vacuum was then reduced and the collection plate (approximately 0.2 ml in each well) was replaced with a new one. This fraction collection process was repeated with 20-75 plates. During this procedure the wells were eluted with mobile phase continuously. Caution was exercised to ensure that the level of buffer in each well did not fall below the surface level of gel. The eluates in the plates were transferred carefully by automatic pipettes and weighed to estimate the retention volume. All procedures were performed at room temperature (20°C). Eluted substances were detected by UV-scans at 230 nm. Chromatograms were obtained by plotting UV absorbance versus fraction volume.

## Claims

1. A method of identifying candidate drug molecules exhibiting week affinity by screening a biological target for transient weak interactions between the target and a library of ligands comprising the steps of:
(i) providing a composition of a biological target;
(ii) providing a plurality of stationary phases from said composition by immobilizing a proteinaceous target to a solid support in form of functionalized silica particles packaged into chromatography wells;
(iii) transporting a plurality of ligand compositions to said stationary phases, thereby establishing contacts between said ligands and said targets;
(iv) collecting, downstream of said stationary phases, zonal retardation information for each ligand and quantitatively detecting said ligands in compositions arriving from said stationary phases to discriminate between different ligand affinities in order to estimate the affinity and the dynamics of each ligand/target interaction; and
(v) selecting ligands exhibiting weak affinity to said target, wherein said ligands have dissociation constants (K_{d}) in the range of 0.01 to 10 mM.

2. A method according to claim 1, wherein the zonal retardation information is selected among retention time and bandwidth.

3. A method according to claim 1 comprising continuously eluting said stationary phase with a mobile phase.

## Patentansprüche

1. Verfahren zum Identifizieren von potentiellen Wirkstoffmolekülen, welche eine schwache Affinität aufweisen, durch Screening eines biologischen Targets auf transiente schwache Wechselwirkungen zwischen dem Target und einer Ligandenbibliothek, umfassend die Schritte:
(i) Bereitstellen einer Zusammensetzung eines biologischen Targets;
(ii) Bereitstellen einer Vielzahl stationärer Phasen aus der Zusammensetzung durch Immobilisieren eines proteinösen Targets an einen festen Träger in Form von funktionalisierten Kieselsäurepartikeln, welche in Chromatographiebehältnissen gepackt sind;
(iii) Transportieren einer Vielzahl von Ligandenzusammensetzungen zu den stationären Phasen, wobei Kontakte zwischen den Liganden und den Targets ausgebildet werden;
(iv) Sammeln stromabwärts von den stationären Phasen von zonalen Retardationsinformationen für jeden Liganden und quantitatives Nachweisen der Liganden in Zusammensetzungen, die von den stationären Phasen eintreffen, um zwischen verschiedenen Ligandenaffinitäten zu unterscheiden, um die Affinität und die Dynamik einer jeden Ligand/Target-Wechselwirkung abzuschätzen; und
(v) Auswählen von Liganden, die eine schwache Affinität für das Target aufweisen, wobei die Liganden Dissoziationskonstanten (K_{d}) im Bereich von 0,01 bis 10 mM haben.

2. Verfahren nach Anspruch 1, wobei die zonalen Retardationsinformationen ausgewählt sind aus Retentionszeit und Bandbreite.

3. Verfahren nach Anspruch 1, umfassend kontinuierliches Eluieren der stationären Phase mit einer mobilen Phase.

## Revendications

1. Procédé d'identification de molécules de médicament candidates présentant une faible affinité en criblant une cible biologique pour des interactions faibles transitoires entre la cible et une bibliothèque de ligands comprenant les étapes de :
(i) fourniture d'une composition d'une cible biologique ;
(ii) fourniture d'une pluralité de phases immobiles à partir de ladite composition en immobilisant une cible protéique sur un support solide en forme de particules de silice fonctionnalisées empaquetées dans des alvéoles de chromatographie ;
(iii) transport d'une pluralité de compositions de ligand vers lesdites phases immobiles, établissant de ce fait des contacts entre lesdits ligands et lesdites cibles ;
(iv) collecte, en aval desdites phases immobiles, d'informations de retardement zonal pour chaque ligand et quantitativement de détection desdits ligands dans des compositions se produisant à partir desdites phases immobiles pour faire la distinction entre des affinités de ligand différentes afin d'évaluer l'affinité et la dynamique de chaque interaction de ligand / cible ; et
(v) sélection de ligands présentant une faible affinité à ladite cible, dans laquelle lesdits ligands ont des constantes de dissociation (K_{d}) dans la plage de 0,01 à 10 mM.

2. Procédé selon la revendication 1, dans lequel les informations de retardement zonal sont sélectionnées parmi le temps de rétention et la bande passante.

3. Procédé selon la revendication 1, comprenant l'élution en continu de ladite phase immobile avec une phase mobile.
